# FASCICULE DE BREVET EUROPEEN

(11) **EP 0 971 233 B1**
(45) Date de publication et mention de la délivrance du brevet: **09.10.2002**
(21) Numéro de dépôt: 99430012.7
(22) Date de dépôt: 05.07.1999
(51) Int. Cl.: G01N 33/50, G01N 33/72

(54) **Réactif et méthode pour la perméabilisation et l'identification des érythrocytes**
Reagenz und Methode zur Permeabilisierung und zur Identifizierung von Erythrozyten
Reagent and method for the permeabilisation and identification of erythrocytes

(30) Priorité: 09.07.1998 FR 9809006
(43) Date de publication de la demande: 12.01.2000
(73) Titulaire: Société Anonyme dite: IMMUNOTECH S.A., F-13276 Marseille Cédex 9 (FR)
(72) Inventeur: Van Agthoven, André, 13009 Marseille (FR); Fornelli, Christine, 13009 Marseille (FR)
(74) Mandataire: Rinuy, Santarelli

(56) Documents cités:
- EP-A- 0 469 766
- EP-A- 0 625 706

## Description

La présente invention concerne de nouveaux réactifs et un nouveau procédé de perméabilisation des érythrocytes, ainsi que son application à l'identification des érythrocytes foetaux par une nouvelle méthode d'immunomarquage.

Des méthodes d'analyse cytométrique des érythrocytes foetaux après marquage à l'aide d'anticorps fluorescents à l'intérieur des érythrocytes existent déjà.

Un anticorps et sa méthode est commercialisé par la société BIOATLANTIQUE à Nantes (France) sous les dénominations « Anticorps monoclonal anti-hémoglobine foetale » et « Protocole pour la cytométrie de flux ». Cette méthode a l'inconvénient d'être longue et compliquée avec douze lavages et 16 heures d'incubation.

Un autre anticorps et méthode est commercialisé par CALTAG LABORATORIES à Burlingame, Californie (USA) sous les dénominations « Monoclonal antibodies to human fetal hemoglobin » et « Anti-HbF-flow cytometric protocol ». Cette méthode est plus courte avec six lavages, mais utilise comme fixateur l'aldéhyde glutarique qui n'est pas stable à température ambiante.

Il serait donc souhaitable de disposer de réactifs pour la perméabilisation érythrocytaire qui soient stables et pourraient être conservés pendant plus d'un an, et d'un procédé de perméabilisation érythrocytaire qui ne comprenne pas plus de quatre étapes de lavage et une incubation courte, par exemple de trente minutes.

Or, après de longues recherches la demanderesse a découvert qu'une perméabilisation des érythrocytes, sans perte substantielle de l'hémoglobine de la cellule, peut être obtenue par le traitement des érythrocytes à l'aide d'un agent de fixation et d'un agent de perméabilisation.

C'est pourquoi la présente demande a pour objet un procédé de perméabilisation des érythrocytes caractérisé en ce que l'on soumet les érythrocytes à l'action successivement
- (a) d'un agent de fixation comprenant un aldéhyde aliphatique et un oligosaccharide,
- (b) d'un agent de perméabilisation comprenant un détergent et un oligosaccharide.

Ce nouveau procédé permet l'entrée d'un anticorps dans l'érythrocyte et l'analyse d'un antigène intracellulaire. Les antigènes présents dans l'érythrocyte et notamment ceux qui sont portés par l'hémoglobine sont utilisés pour la distinction et l'identification des érythrocytes foetaux.

L'agent de fixation contient un aldéhyde aliphatique, un oligosaccharide et de préférence un polysaccharide sulfaté. Il est notamment dans un milieu environ isotonique ou hypertonique et de préférence légèrement acide.

L'aldéhyde aliphatique de préférence en C1-C5 peut être par exemple le paraformaldéhyde et particulièrement l'aldéhyde formique.

L'aldéhyde aliphatique peut être présent dans une concentration de 0,3M à 12,3M, particulièrement de 1,5M à 10M et tout particulièrement de 3M à 8,3M. Dans des conditions préférentielles de mise en oeuvre de l'agent de fixation ci-dessus, on utilise 6,7M de l'aldéhyde formique.

L'agent de fixation contient également un oligosaccharide, de préférence un disaccharide. On peut citer par exemple, à titre de disaccharide, le saccharose, le cellobiose, le maltose, le lactose, le gentiobiose, le mélibiose et particulièrement le tréhalose.

L'oligosaccharide peut être présent à une concentration de 0, 025M à 1,32M, particulièrement de 0,132M à 1,19M, et tout particulièrement de 0,26M à 1,06M. Dans des conditions préférentielles, l'agent de fixation contient environ 0,8M de tréhalose.

Un polysaccharide sulfaté est avantageusement utilisé pour éviter des agrégations incluant des acides nucléiques, ce qui permet de diminuer le bruit de fond lorsque le procédé ci-dessus est utilisé dans un procédé de détection des érythrocytes.

Il se trouve dans l'agent de fixation à une concentration se situant entre 0,1 mg/ml et 10 mg/ml. De préférence, l'agent de fixation contient environ 1 mg/ml de sulfate de dextran à un poids moléculaire autour de 500.000.

Le pH de l'agent de fixation est par exemple un pH entre 3 et 8, particulièrement entre 4 et 7. Il est de préférence légèrement acide, tout particulièrement entre 5 et 6. L'agent de fixation dans des conditions particulières a un pH de 5,5 environ, de préférence tamponné par une solution 10mM de dihydrogéno-phosphate de sodium.

L'isotonicité de l'agent de fixation est obtenue de préférence par la présence d'une concentration de 0,15M NaCI.

L'agent de perméabilisation contient un détergent de préférence zwitter-ionique, et particulièrement un détergent anionique. Ce détergent anionique est par exemple du dioxycholate de sodium ou N-lauroyl sarcoside, et particulièrement du dodécyl sulfate de sodium.

La concentration du détergent peut être entre 0,001% et 10%, et particulièrement entre 0,01 et 5%. Dans des conditions préférentielles, l'agent de perméabilisation contient environ 0,03% de dodécyl sulfate de sodium.

L'agent de perméabilisation contient en outre un oligosaccharide, particulièrement le tréhalose comme décrit dans le cas de l'agent de fixation.

La concentration de tréhalose dans le tampon de perméabilisation se situe par exemple entre 0,0026M et 0,26M, de préférence entre 0,026M et 0,13M, et tout particulièrement à environ 0,053M.

Le pH de l'agent de perméabilisation est de préférence légèrement acide avec un pH entre 3 et 8, particulièrement entre 5 et 6. L'agent de perméabilisation dans des conditions particulières a un pH de 5,5 environ.

Le pH de l'agent de perméabilisation est de préférence tamponné, avantageusement par utilisation de 10mM de dihydrogénophosphate de sodium et 10mM de citrate de sodium.

Le tampon de perméabilisation est de préférence environ isotonique, par exemple par utilisation d'une concentration de 0,15M de chlorure de sodium.

Le procédé de l'invention trouve son application notamment dans l'identification des érythrocytes foetaux par une nouvelle méthode d'immunomarquage utilisant des anticorps. C'est pourquoi, après fixation et perméabilisation, les érythrocytes sont avantageusement lavés pour principalement éliminer le détergent qui pourrait avoir une influence néfaste sur les anticorps utilisés pour la mise en évidence des antigènes à l'intérieur de l'érythrocyte.

L'agent de lavage est de préférence une solution de pH entre 3 et 8, et avantageusement légèrement acide, entre pH 5 et 6. Cette solution peut contenir un détergent neutre et peut contenir un sel d'acide et base forts comme NaCl et KCI. De préférence, l'agent de lavage ne contient pas de détergent et est hypotonique. Dans les conditions particulièrement préférées, l'agent de lavage est de l'eau distillée ou déminéralisée.

La présente demande a également pour objet un nouveau procédé pour mettre en évidence des érythrocytes foetaux parmi une population d'érythrocytes d'un sang adulte.

La méthode la plus connue est celle de Kleihauer basée sur une meilleure résistance des érythrocytes foetaux contre une lyse hypotonique. Sous certaines conditions hypotoniques, un relargage de l'hémoglobine des érythrocytes adultes est observé, mais pas des érythrocytes foetaux, ce qui permet de distinguer les érythrocytes foetaux à base de la couleur par inspection microscopique.

L'inconvénient de cette méthode est que pour un faible nombre d'érythrocytes foetaux à détecter, un grand nombre d'érythrocytes devait passer au microscope, ce qui représente une manipulation importante. Aussi, certaines pathologies peuvent conduire à une modification des érythrocytes, entraînant un comptage de faux positifs. De plus, selon certains rapports, ce test serait peu reproductible.

D'autres méthodes sont basées sur la perméabilisation des érythrocytes et immunomarquage de l'hémoglobine foetale (g-hémoglobine) à l'intérieur des érythrocytes foetaux avec un anticorps fluorescent. La dernière méthode permet un comptage par cytométrie mais elle a comme inconvénient que certains érythrocytes d'origine adulte peuvent contenir de l'hémoglobine foetale (F cells). Egalement, dans certaines maladies comme la Thalassémie, le nombre d"érythrocytes adultes contenant l'hémoglobine foetale est augmenté.

Il serait donc souhaitable de disposer d'un procédé d'immuno-marquage des érythrocytes foetaux de haute sensibilité et possibilités d'erreur réduites.

L'invention a donc également pour objet un procédé de perméabilisation ci-dessus, caractérisé en ce que en outre, (d) l'on fait réagir les érythrocytes avec deux anticorps contre deux antigènes foetaux qui sont exprimés sur l'érythrocyte de façon indépendante. L'une, l'hémoglobine foetale se trouve à l'intérieur de la cellule, l'autre, l'antigène i, à l'extérieur de la cellule.

Au cours de cette étape d'immunomarquage, les érythrocytes, de préférence en suspension dans l'eau, sont avantageusement mélangés avec un volume égal d'un tampon phosphate contenant 5 mg/ml de Sérum Albumine Bovine et les deux anticorps. Lors de cette étape, le pH devient neutre, permettant un bon fonctionnement des anticorps et l'environnement de l'érythrocyte reste légèrement hypotonique, permettant une bonne pénétration des anticorps.

L'immunomarquage simultané de l'hémoglobine foetale et i avec des anticorps marqués avec deux marqueurs fluorescents différents permet un comptage des érythrocytes foetaux les distinguant les « F-cells ».

Le marquage des anticorps avec les marqueurs fluorescents comme le N-isothiocyanate de fluorescéine ou la phycoérythrine par exemple peut être réalisé de manière conventionnelle comme décrit par exemple par G.T. HERMANSON dans Bioconjugate Techniques, Chapitre 8.1.1, Fluorescein Derivatives pages 302-305, Academic Press 1996 ou Chapitre 8.1.7., Phycobiliprotein Derivatives pages 362-364. On peut notamment conjuguer l'anticorps désiré à la phycoérythrine activée avec du succinimidyl-4-(N-maléimidométhyl)cyclohexane-1-carboxylate après réduction avec 1mM de DL-dithiothreitol en PBS.

Dans des conditions préférentielles de mise en oeuvre du procédé ci-dessus décrit, on utilise pour marquer l'hémoglobine foetale l'anticorps monoclonal de type IgG1 NaM16-2F4 déposé à la Collection Nationale de Microorganismes (CNCM) à Paris le 23 juin 1998 sous le N° I-2044 et pour marquer l'antigène i, l'anticorps monoclonal de type IgM NaM61-1A2 déposé à la Collection Nationale de Microorganismes (CNCM) à Paris le 23 juin 1998 sous le N° I-2043.

Dans d'autres conditions préférentielles de mise en oeuvre de l'invention, une augmentation de la sensibilité du test est obtenue en rajoutant un marqueur des acides nucléiques, d'une troisième couleur.

En cytométrie en trois couleurs, on peut ainsi éliminer des événements correspondant à un faux « bruit de fond », souvent des leucocytes ou des cellules mortes qui sont riches en ADN.

Dans des conditions particulièrement préférées, le marqueur des acides nucléiques utilisé est un colorant des cellules nuclées, par exemple par fixation sur l'ADN, particulièrement le LDS 751 (MOLECULAR PROBES, Inc. Or, USA).

La présente demande a encore pour objet un kit de perméabilisation des érythrocytes, caractérisé en ce qu'il renferme
- (a) un agent de fixation environ isotonique comprenant un aldéhyde aliphatique et un oligosaccharide,
- (b) un agent de perméabilisation comprenant un détergent et un oligosaccharide.

La présente demande a encore pour objet un kit d'immuno-marquage des érythrocytes foetaux, caractérisé en ce qu'il comprend un kit de perméabilisation des érythrocytes ci-dessus ainsi que deux anticorps contre deux antigènes foetaux qui sont exprimés sur l'érythrocyte de façon indépendante.

La présente demande a enfin pour objet un procédé d'identification des érythrocytes foetaux par immunomarquage, caractérisé en ce que l'on soumet les érythrocytes à l'action successivement :
- (a) d'un agent de fixation comprenant un aldéhyde aliphatique et un oligosaccharide dans un milieu environ isotonique, ou hypertonique,
- (b) d'un agent de perméabilisation comprenant un détergent et un oligosaccharidel'on opère selon la revendication 8
- (c) d'un agent de lavage pour principalement éliminer le détergent
(d) de deux anticorps contre deux antigènes foetaux qui sont exprimés sur l'érythrocyte de façon indépendante
puis procède à la détection des érythrocytes foetaux.

Cette détection peut être réalisée par tous les moyens bien connus de l'état de la technique de détection de composés fluorescents, combinée à l'utilisation de seuils de mesures ou de cadres de mesures.

Les conditions préférentielles de mise en oeuvre des procédés ci-dessus décrites s'appliquent également aux autres objets de l'invention visés ci-dessus, notamment les kits.

Les exemples qui suivent illustrent la présente demande.

### Exemple 1 : Réactif de fixation

6,67M aldéhyde formique
0,8M D (+) Tréhalose
0,15M Chlorure de sodium
0,01M dihydrogénophosphate de sodium
1 mg/ml dextran sulfate (PM 500.000)
pH 5,5

### Réactif de perméabilisation

0,001 M dodécyl sulfate de sodium
0,15M NaCl
0,01M dihydrogéno phosphate de sodium
0,01M citrate de sodium
0,053M D(+) Tréhalose
pH 5,5

### Exemple 2 : procédure de perméabilisation

Quatre échantillons sanguins ont été choisis pour être soumis à une procédure de perméabilisation.

Les échantillons ont été utilisés avec de l'Acide éthylène diamine tétraacétique (EDTA) comme anticoagulant.
1. Sang d'un sujet mâle.
2. Un millilitre du même sang décrit en 1 auquel il est rajouté 0,01 ml de sang de cordon ombilical pris pendant la naissance d'un enfant.
3. Sang d'une femme enceinte, dont le sang a été inspecté par la méthode Kleihauer et pour lequel le résultat rendu était négatif.
4. Sang d'une femme enceinte dont le sang a été inspecté par la méthode Kleihauer et pour lequel le résultat rendu était positif, donnant une valeur de 0,1% d'érythrocytes foetaux.

Chaque échantillon de 0,01 ml de sang a été mélangé à 0,1 ml de réactif_de fixation (exemple 1) et le mélange a été incubé pendant 30 minutes à température ambiante (20-25°C). Après incubation, 3 ml de tampon phosphate (PBS = Phosphate Buffered Saline) ont été rajoutés comme lavage et l'ensemble a été centrifugé à 300g.

Après aspiration, les cellules ont été reprises dans 3 ml de réactif de perméabilisation (exemple 1) et centrifugées à 300g.

Après aspiration, les cellules ont été reprises dans 1 ml d'eau déminéralisée.

### Exemple 3 : Immunomarquage des érythrocytes foetaux

20µl de chacune des préparations de l'exemple 2 d'érythrocytes en suspension dans l'eau ont été mélangés à 20 µl de la préparation suivante :

PBS contenant :
- 50µg/ml d'anticorps monoclonal NaM16-2F4 dirigé contre l'hémoglobine foetale et conjugué au fluorescéine isothiocyonate comme décrit par G.T. HERMANSON dans Bioconjugate Techniques, Chapitre 8.1.1, Fluorescein Derivatives pages 302-305, Academic Press 1996;
- 3,12 µg/ml d'anticorps monoclonal NaM61-1A2 dirigé contre l'antigène i et conjugué à la phycoérythrine comme décrit par G.T. HERMANSON dans Bioconjugate Techniques, Chapitre 8.1.7., Phycobiliprotein Derivatives pages 362-364 ;
- 3,12 µg/ml de LDS 751 (Molecular Probes, OR, USA).
- 5 mg/ml de Sérum Albumine Bovine (BSA).

Après une incubation de 15 minutes, 3 ml de tampon phosphate contenant 0,17M d'aldéhyde formique ont été rajoutés à chaque mélange comme lavage et centrifugés à 300g pendant 5 minutes. Après aspiration, les cellules ont été reprises dans 0,5 ml de tampon phosphate, 0, 17M d'aldéhyde formique.

L'hybridome correspondant à l'anticorps monoclonal NaM16-2F4 a été déposé à la Collection Nationale de Microorganismes (CNCM) à Paris le 23 juin 1998 sous le N° I-2044. L'anticorps monoclonal NaM16-2F4 est de type IgG1. L'hybridome correspondant à l'anticorps monoclonal NaM61-1A2 a été déposé à la Collection Nationale de Microorganismes (CNCM) à Paris 23 juin 1998 sous le N° I-2043. L'anticorps monoclonal NaM61-1A2 est de type IgM.

### Exemple 4 : Analyse cytométrique

Un cytomètre Coulter XL (Miami, Floride, USA) a été utilisé pour l'analyse des résultats. Les réglages de fluorescence FL1 et FL2 et les compensations ont été obtenus selon les instructions du fabricant en utilisant un échantillon de référence « Cytotrol » (Coulter N° Cat 6604248), mélangé avec 20µl de l'échantillon N°1 de l'exemple 2, et marqué avec un mélange de CD4-FITC(N-isothiocyanate de fluorescéïne) et CD8-PE (phycoérythrine) (lmmunotech, N° Cat IM0747).

Le réglage et la compensation de FL4 ont été obtenus avec l'échantillon 2 de l'exemple 2, après immunomarquage selon l'exemple 3.

La figure 1 montre un scattergramme des événements de l'échantillon 2 (exemple 2) après immunomarquage (exemple 3). Les événements de taille inférieure à celle des érythrocytes ont été exclus par un seuil.

La figure 2 montre une analyse en diffusion aux grands angles et FL4 des événements de l'échantillon 2 (exemple 2) après immunomarquage (exemple 3) et exclusion des événements de taille inférieure comme montré en figure 1. Les événements positifs en LDS 751 (leucocytes et particules non identifiés) ont été mis en dehors d'un cadre.

La figure 3 montre une analyse en FL1 et FL2 sur des populations érythrocytaires après immunomarquage (exemple 3) et incluses dans un cadre comme montré en figure 2.

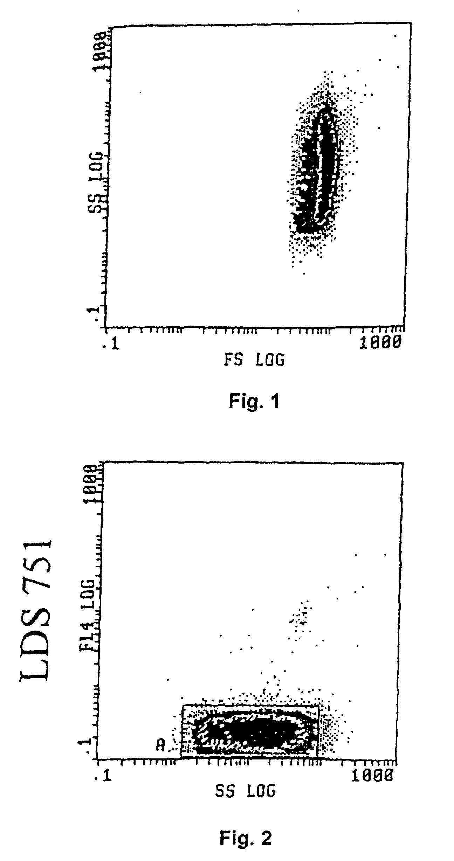

Sur la figure 1, l'on peut observer une population d'évènements homogènes d'érythrocytes adultes et foetaux. Un seuil dans la diffusion aux petits angles exclut les évènements d'une taille inférieure au seuil choisi tels que ceux correspondant aux plaquettes et débris. Un faible nombre de leucocytes et de débris de grande taille sont inclus dans la population.

Sur la figure 2, l'on peut observer la même population que dans la figure 1 représentée en diffusion aux grands angles et en FL4, région de fluorescence émise par le LDS751. Les événements positifs pour le LDS751, voire les leucocytes et les débris de grande taille, sont exclus de l'analyse par un cadre créé autour des événements négatifs y compris les érythrocytes adultes et foetaux.

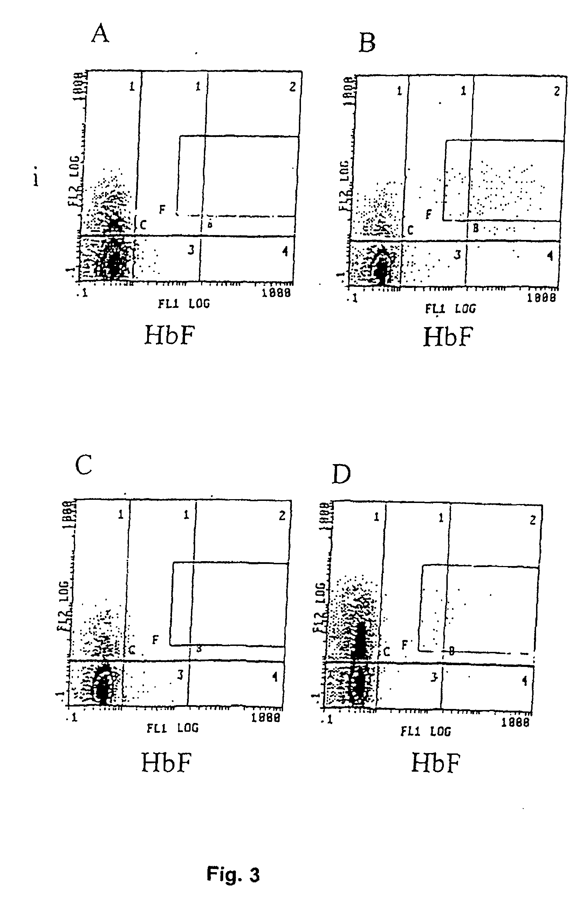

Sur la figure 3, où les échantillons 1, 2, 3, 4 (Echantillon 1 (exemple 2): érythrocytes d'un sang d'adulte servant de contrôle ; Echantillon 2 (exemple 2): érythrocytes d'un sang adulte mélangé avec 0,01 v/v d'un sang de cordon ; Echantillon 3 (exemple 2) : érythrocytes d'un sang Kleihauer négatif servant de contrôle ; Echantillon 4 (exemple 2): érythrocytes d'un sang Kleihauer positif ) correspondent respectivement à A, B, C, et D, l'on peut observer une analyse en FL1 et FL2 des populations d'événements comme représenté dans le cadre en figure 2. La figure 3B correspond effectivement à la représentation de la figure 2, ce qui est similaire aux représentations correspondant à la figure 3A, C et D.

Dans les figures, le FL1 correspond à l'expression de l'hémoglobine foetale, et le FL2 à l'expression de i. La bande de gauche définie par l'abcisse = 1 contient les événements correspondant aux érythrocytes adultes. et l'on remarque une distinction nette entre les érythrocytes adultes et foetaux. Dans la figure 3B, on observe dans la fenêtre F des événements n'existant pas dans la figure 3A et correspondant au sang de cordon qui a été rajouté au sang adulte. Dans la figure 3D, on observe dans la fenêtre F des événements n'existant pas dans la figure 3C et correspondant aux érythrocytes foetaux qui ont circulé dans le sang d'une femme après hémorragie foetale.

On peut conclure de ce qui précède que la méthode et les réactifs de détection des hématies foetales de l'invention ont fait preuve d'efficacité dans un modèle où le sang de cordon a été rajouté à un sang adulte et également dans les cas d'hémorragie foetale de la femme enceinte.

On peut également conclure de ce qui précède que la méthode et les réactifs ci-dessus permettent la détection de la chaîne gamma de l'hémoglobine dans l'hématie. Outre les cas d'hémorragie foetale de la femme enceinte la méthode peut s'appliquer notamment dans des cas de thalassémie, de maladie des hématies cruciformes, de carence de fer chez la femme enceinte, etc. La méthode et les réactifs permettent également de détecter par la présence ou non du groupe sanguin i, la provenance foetale d'une hématie ou non.

## Revendications

1. Un procédé de perméabilisation des érythrocytes dans un échantillon **caractérisé en ce que** l'on soumet les érythrocytes à l'action successivement
- (a) d'un agent de fixation comprenant un aldéhyde aliphatique et un oligosaccharide,
- (b) d'un agent de perméabilisation comprenant un détergent et un oligosaccharide.

2. Un procédé de perméabilisation selon la revendication 1, **caractérisé en ce que** l'agent de fixation est dans un milieu environ isotonique, ou hypertonique.

3. Un procédé de perméabilisation selon l'une des revendications 1 et 2, **caractérisé en ce que** l'agent de fixation a un pH de 4 à 7.

4. Un procédé de perméabilisation selon l'une des revendications 1 à 3, **caractérisé en ce que** le détergent de l'agent de perméabilisation est anionique.

5. Un procédé de perméabilisation selon l'une des revendications 1 à 4, **caractérisé en ce que** l'agent de perméabilisation a un pH de 5 à 6.

6. Un procédé de perméabilisation selon l'une des revendications 1 à 5, **caractérisé en ce que** en outre, (c) après fixation et perméabilisation, les érythrocytes sont lavés à l'aide d'un agent de lavage pour principalement éliminer le détergent.

7. Un procédé de perméabilisation l'une des revendications 1 à 6, **caractérisé en ce que** l'agent de fixation comprend en outre un polysaccharide sulfaté.

8. Un procédé de perméabilisation selon l'une des revendications 1 à 7, **caractérisé en ce que** en outre, (d) l'on fait réagir les érythrocytes avec deux anticorps contre deux antigènes foetaux qui sont exprimés sur l'érythrocyte de façon indépendante.

9. Un kit de perméabilisation des érythrocytes, **caractérisé en ce qu'**il renferme
- (a) un agent de fixation environ isotonique, ou hypertonique, comprenant un aldéhyde aliphatique et un oligosaccharide,
- (b) un agent de perméabilisation comprenant un détergent et un oligosaccharide.

10. Un kit d'immuno-marquage des érythrocytes foetaux, **caractérisé en ce qu'**il comprend un kit de perméabilisation des érythrocytes selon la revendication 9 ainsi que deux anticorps contre deux antigènes foetaux qui sont exprimés sur l'érythrocyte de façon indépendante.

11. Un kit selon la revendication 10, **caractérisé en ce que** les deux anticorps contre deux antigènes foetaux qui sont exprimés sur l'érythrocyte de façon indépendante sont marqués avec deux marqueurs fluorescents différents.

12. Un kit selon la revendication 10 ou 11, **caractérisé en ce que** les deux antigènes foetaux qui sont exprimés sur l'érythrocyte de façon indépendante sont l'un, l'hémoglobine foetale qui se trouve à l'intérieur de la cellule, l'autre, l'antigène i, qui se trouve à l'extérieur de la cellule.

13. Un kit selon l'une des revendications 10 à 12, **caractérisé en ce que** l'on utilise pour marquer l'hémoglobine foetale, l'anticorps NaM16-2F4 monoclonal de type IgG1 déposé à la Collection Nationale de Microorganismes (CNCM) à Paris le 23 juin 1998 sous le N° I-2044, et pour marquer l'antigène i l'anticorps monoclonal NaM61-1A2 de type IgM déposé à la Collection Nationale de Microorganismes (CNCM) à Paris le 23 juin 1998 sous le N° I-2043.

14. Un procédé d'identification des érythrocytes foetaux dans un échantillon par immunomarquage, **caractérisé en ce que** l'on soumet les érythrocytes à l'action successivement
- (a) d'un agent de fixation comprenant un aldéhyde aliphatique et un oligosaccharide dans un milieu environ isotonique, ou hypertonique,
- (b) d'un agent de perméabilisation comprenant un détergent et un oligosaccharide
- (c) d'un agent de lavage pour principalement éliminer le détergent
(d) de deux anticorps contre deux antigènes foetaux qui sont exprimés sur l'érythrocyte de façon indépendante
puis procède à la détection des érythrocytes foetaux.

## Claims

1. A process for permeabilizing erythrocytes in a sample **characterized in that** the erythrocytes are subjected successively to the action of
- (a) a fixing agent containing an aliphatic aldehyde and an oligosaccharide,
- (b) a permeabilizing agent containing a detergent and an oligosaccharide.

2. A permeabilization process according to claim 1, **characterized in that** the fixing agent is in an approximately isotonic or hypertonic medium.

3. A permeabilization process according to one of claims 1 and 2, **characterized in that** the fixing agent has a pH of 4 to 7.

4. A permeabilization process according to one of claims 1 to 3, **characterized in that** the detergent of the permeabilization agent is anionic.

5. A permeabilization process according to one of claims 1 to 4, **characterized in that** the permeabilizing agent has a pH of 5 to 6.

6. A permeabilization process according to one of claims 1 to 5, **characterized in that** in addition, (c) after fixation and permeabilization, the erythrocytes are washed using a washing agent mainly in order to eliminate the detergent.

7. A permeabilization process according to one of claims 1 to 6, **characterized in that** the fixing agent also contains a sulphated polysaccharide.

8. A permeabilization process according to one of claims 1 to 7, **characterized in that** in addition, (d) the erythrocytes are reacted with two antibodies against two foetal antigens which are expressed independently on the erythrocyte.

9. A kit for permeabilizing erythrocytes, **characterized in that** it contains
- (a) an approximately isotonic or hypertonic fixing agent containing an aliphatic aldehyde and an oligosaccharide,
- (b) a permeabilizing agent containing a detergent and an oligosaccharide.

10. A kit for immuno-marking foetal erythrocytes, **characterized in that** it contains a kit for permeabilizing erythrocytes according to claim 9 as well as two antibodies against two foetal antigens which are expressed independently on the erythrocyte.

11. A kit according to claim 10, **characterized in that** the two antibodies against two foetal antigens which are expressed independently on the erythrocyte are marked with two different fluorescent markers.

12. A kit according to claim 10 or 11, **characterized in that** the two foetal antigens which are expressed independently on the erythrocyte are, on the one hand, foetal haemoglobin found inside the cell, and on the other hand, the antigen i found on the outside of the cell.

13. A kit according to one of claims 10 and 12, **characterized in that** the monoclonal antibody NaM16-2F4 of IgG1 type deposited at the Collection Nationale de Microorganismes (CNCM) in Paris on 23^{rd} June 1998 under No. I-2044 is used for marking the foetal haemoglobin, and monoclonal antibody NaM61-1A2 of IgM type deposited at the Collection Nationale de Microorganismes (CNCM) at Paris on 23^{rd} June 1998 under No. I-2043 is used for marking the antigen i.

14. A process for identifying foetal erythrocytes in a sample by immuno-marking, **characterized in that** the erythrocytes are subjected successively to the action of
- (a) a fixing agent containing an aliphatic aldehyde and an oligosaccharide in an approximately isotonic or hypertonic medium,
- (b) a permeabilizing agent containing a detergent and an oligosaccharide
- (c) a washing agent mainly in order to eliminate the detergent
- (d) two antibodies against two foetal antigens which are expressed independently on the erythrocyte
then the foetal erythrocytes are detected.

## Patentansprüche

1. Ein Verfahren zur Permeabilisierung von Erythrozyten in einer Probe, **dadurch gekennzeichnet, dass** man die Erythrozyten sukzessiv der Wirkung
(a) eines Fixierungsagens, umfassend einen aliphatischen Aldehyd und ein Oligosaccharid,
(b) eines Permeabilisierungsagens, umfassend ein Detergens und ein Oligosaccharid
aussetzt.

2. Ein Verfahren zur Permeabilisierung gemäß Anspruch 1, **dadurch gekennzeichnet, dass** das Fixierungsagens in einem ungefähr isotonischen oder hypertonischen Milieu ist.

3. Ein Verfahren zur Permeabilisierung gemäß einem der Ansprüche 1 und 2, **dadurch gekennzeichnet, dass** das Fixierungsagens einen pH von 4 bis 7 aufweist.

4. Ein Verfahren zur Permeabilisierung gemäß einem der Ansprüche 1 bis 3, **dadurch gekennzeichnet, dass** das Detergens des Permeabilisierungsagens anionisch ist.

5. Ein Verfahren zur Permeabilisierung gemäß einem der Ansprüche 1 bis 4, **dadurch gekennzeichnet, dass** das Permeabilisierungsagens einen pH von 5 bis 6 aufweist.

6. Ein Verfahren zur Permeabilisierung gemäß einem der Ansprüche 1 bis 5, **dadurch gekennzeichnet, dass** weiters (c) nach Fixierung und Permeabilisierung die Erythrozyten mit Hilfe eines Waschagens gewaschen werden, um das Detergens im Wesentlichen zu entfernen.

7. Ein Verfahren zur Permeabilisierung gemäß einem der Ansprüche 1 bis 6, **dadurch gekennzeichnet, dass** das Fixierungsagens weiters ein sulfatiertes Polysaccharid umfasst.

8. Ein Verfahren zur Permeabilisierung nach einem der Ansprüche 1 bis 7, **dadurch gekennzeichnet, dass** weiters (d) die Erythrozyten mit zwei Antikörpern gegen zwei fötale Antigene, die auf unabhängige Weise auf dem Erythrozyten exprimiert sind, in Reaktion gebracht werden.

9. Ein Kit zur Permeabilisierung von Erythrozyten, **dadurch gekennzeichnet, dass** er umfasst:
(a) ein ungefähr isotonisches oder hypertonisches Fixierungsagens, umfassend einen aliphatischen Aldehyd und ein Oligosaccharid,
(b) ein Permeabilisierungsagens, umfassend ein Detergens und ein Oligosaccharid.

10. Ein Kit zur Immunomarkierung von fötalen Erythrozyten, **dadurch gekennzeichnet, dass** er einen Kit zur Permeabilisierung von Erythrozyten gemäß Anspruch 9 sowie zwei Antikörper gegen zwei fötale Antigene, die auf unabhängige Weise auf dem Erythrozyten exprimiert sind, umfasst.

11. Ein Kit gemäß Anspruch 10, **dadurch gekennzeichnet, dass** die zwei Antikörper gegen zwei fötale Antigene, die auf unabhängige Weise auf dem Erythrozyten exprimiert sind, mit zwei verschiedenenen fluoreszierenden Markern markiert sind.

12. Ein Kit gemäß Anspruch 10 oder 11, **dadurch gekennzeichnet, dass** die zwei fötalen Antigene, die auf unabhängige Weise auf dem Erythrozyten exprimiert sind, erstens ein fötales Hämoglobin, das sich im Inneren der Zelle befindet, und zweitens das Antigen i, das sich außerhalb der Zelle befindet, sind.

13. Ein Kit gemäß einem der Ansprüche 10 bis 12, **dadurch gekennzeichnet, dass** für die Markierung des fötalen Hämoglobins der monoklonale Antikörper NaM16-2F4 des Typs IgG1, hinterlegt bei der Nationalen Sammelstelle für Mikroorganismen (CNCM) in Paris am 23. Juni 1998 unter der Nummer I-2044, und zur Markierung des Antigens i der monoklonale Antikörper NaM61-1A2 des Typs IgM, hinterlegt bei der Nationalen Sammelstelle für Mikroorganismen (CNCM) in Paris am 23. Juni 1998 unter der Nummer I-2043, verwendet wird.

14. Ein Verfahren zur Identifizierung von fötalen Erythrozyten in einer Probe mittels Immunomarkierung, **dadurch gekennzeichnet, dass** man die Erythrozyten sukzessiv der Wirkung
(a) eines Fixierungsagens, umfassend einen aliphatischen Aldehyd und ein Oligosaccharid in einem ungefähr isotonischen oder hypertonischen Milieu,
(b) eines Permeabilisierungsagens, umfassend ein Detergens und ein Oligosaccharid,
(c) eines Waschagens, um das Detergens im Wesentlichen zu entfernen,
(d) zweier Antikörper gegen zwei fötale Antigene, die auf unabhängige Weise auf dem Erythrozyten exprimiert sind,
aussetzt, und anschließend die Detektion der fötalen Erythrozyten durchführt.
